# EUROPÄISCHE PATENTSCHRIFT

(11) **EP 0 758 647 B1**
(45) Veröffentlichungstag und Bekanntmachung des Hinweises auf die Patenterteilung: **02.11.2000**
(21) Anmeldenummer: 96112426.0
(22) Anmeldetag: 01.08.1996
(51) Int. Cl.: C07D 309/32, A61K 31/35

(54) **Acyl-substituierte Aminopyrane mit einer modulierende Wirkung auf Kaliumkanäle**
Acyl substituted aminopyrans with a modulating effect on calcium channels
Aminopyranes substitués par un groupe acyl modulant le canal à calcium

(30) Priorität: 14.08.1995 DE 19529859
(43) Veröffentlichungstag der Anmeldung: 19.02.1997
(73) Patentinhaber: BAYER AG, 51368 Leverkusen (DE)
(72) Erfinder: Urbahns, Klaus, Dr., 42115 Wuppertal (DE); Heine, Hans-Georg, Dr., 47800 Krefeld (DE); Junge, Bodo, Dr., 42399 Wuppertal (DE); Mauler, Frank, Dr., 51491 Overath (DE); Wittka, Reilinde, Dr., 51069 Köln (DE); De Vry, Jean-Marie-Viktor, Dr., 51503 Rösrath (DE)

(56) Entgegenhaltungen:
- EP-A- 0 088 276
- WO-A-96/06091
- GB-A- 1 402 793

## Beschreibung

Die vorliegende Erfindung betrifft die Verwendung von Acyl-substituierten Aminopyranen als Arzneimittel, neue Wirkstoffe, ein Verfahren zu ihrer Herstellung, insbesondere ihre Verwendung zur Behandlung von Depression, Psychosen, Sichelzellanämie und Ödemen.

Aus der Publikation Heterocycles (1986), 24 (4), 935-8 sind einige 2-Amino-pyrane als Synthesebausteine bekannt. Außerdem werden in der DE 22 35 406 2-Amino-4H-pyrane mit einer antihypertensiven Wirkung beschrieben.

Es wurde nun gefunden, daß die Acyl-substituierten Aminopyrane der allgemeinen Formel (I), 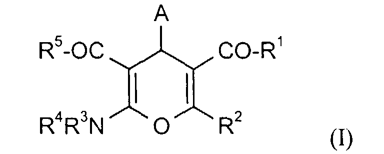 in welcher
- A: für Aryl mit 6 bis 10 Kohlenstoffatomen oder Pyridyl steht, die gegebenenfalls bis zu 3-fach gleich oder verschieden durch Nitro, Cyano, Phenyl, Halogen, Trifluormethyl oder durch geradkettiges oder verzweigtes Alkylthio oder Alkoxy mit jeweils bis zu 6 Kohlenstoffatomen substituiert sind,
- R¹: für geradkettiges oder verzweigtes Alkyl mit bis zu 8 Kohlenstoffatomen steht,
- R²: für geradkettiges oder verzweigtes Alkyl mit bis zu 6 Kohlenstoffatomen steht,
- R³ und R⁴: gleich oder verschieden sind und für Wasserstoff oder für geradkettiges oder verzweigtes Alkyl oder Acyl mit jeweils bis zu 6 Kohlenstoffatomen stehen,
- R⁵: für geradkettiges oder verzweigtes Alkyl oder Alkoxy mit jeweils bis zu 8 Kohlenstoffatomen steht,
und deren Salze
überraschenderweise eine modulierende Wirkung auf Kaliumkanäle besitzen und somit geeignet sind zur Verwendung und Herstellung von Arzneimitteln zur Behandlung von Depression, Psychosen, Sichelzellanämie und Ödemen.

Im Rahmen der Erfindung sind physiologisch unbedenkliche Salze bevorzugt. Physiologisch unbedenkliche Salze sind im allgemeinen Salze der erfindungsgemäßen Verbindungen mit anorganischen oder organischen Säuren. Bevorzugt werden Salze mit anorganischen Säuren wie beispielsweise Salzsäure, Bromwasserstoffsäure, Phosphorsäure oder Schwefelsäure, oder Salze mit organischen Carbon- oder Sulfonsäuren wie beispielsweise Essigsäure, Maleinsäure, Fumarsäure, Äpfelsäure, Zitronensäure, Weinsäure, Milchsäure, Benzoesäure, oder Methansulfonsäure, Ethansulfonsäure, Phenylsulfonsäure, Toluolsulfonsäure oder Naphthalindisulfonsäure.

Die erfindungsgemäßen Verbindungen können in stereoisomeren Formen existieren, die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen.

Bevorzugt sind Verbindungen der allgemeinen Formel (I),
in welcher
- A: für Phenyl, Naphthyl oder Pyridyl steht, die gegebenenfalls bis zu 3-fach gleich oder verschieden durch Nitro, Cyano, Fluor, Chlor, Brom, Iod, Phenyl, Trifluormethyl oder durch geradkettiges oder verzweigtes Alkylthio oder Alkoxy mit jeweils bis zu 4 Kohlenstoffatomen substituiert sind,
- R¹: für geradkettiges oder verzweigtes Alkyl mit bis zu 6 Kohlenstoffatomen steht,
- R²: für geradkettiges oder verzweigtes Alkyl mit bis zu 4 Kohlenstoffatomen steht,
- R³ und R⁴: gleich oder verschieden sind und für Wasserstoff oder für geradkettiges oder für verzweigtes Alkyl oder Acyl mit jeweils bis zu 4 Kohlenstoffatomen stehen,
- R⁵: für geradkettiges oder verzweigtes Alkyl oder Alkoxy mit jeweils bis zu 6 Kohlenstoffatomen steht,
und deren Salze,
bei der Behandlung von Depression, Psychosen, Sichelzellanämie und Ödemen.

Besonders bevorzugt sind Verbindungen der allgemeinen Formel (I),
in welcher
- A: für Phenyl steht, das gegebenenfalls bis zu 3-fach gleich oder verschieden durch Nitro, Cyano, Fluor, Chlor, Brom, Iod, Trifluormethyl oder Methoxy substituiert ist,
- R¹: für Methyl oder Ethyl steht,
- R²: für Methyl oder Ethyl steht,
- R³ und R⁴: gleich oder verschieden sind und für Wasserstoff oder für geradkettiges oder verzweigtes Alkyl oder Acyl mit jeweils bis zu 3 Kohlenstoffatomen stehen,
- R⁵: für geradkettiges oder verzweigtes Alkyl oder Alkoxy mit jeweils bis zu 4 Kohlenstoffatomen steht,
und deren Salze,
bei der Behandlung von Depression, Psychosen, Sichelzellanämie und Ödemen.

Sie sind Modulatoren mit Selektivität für calciumabhängige und Charybdotoxin-sensitive Kalium-Kanäle, insbesondere des zentralen Nervensystems.

Sie sind wertvoll zur Behandlung von Depressionen und Psychosen, z.B. Schizophrenie.

Die Wirkstoffe sind ferner geeignet zur Behandlung von Sichelzellanämie und Ödemen.

Die Erfindung betrifft außerdem neue Verbindungen der Formel (Ia) 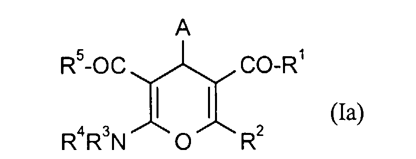 und deren Salze,
mit den in der folgenden Tabelle angegebenen Substituentenbedeutungen:

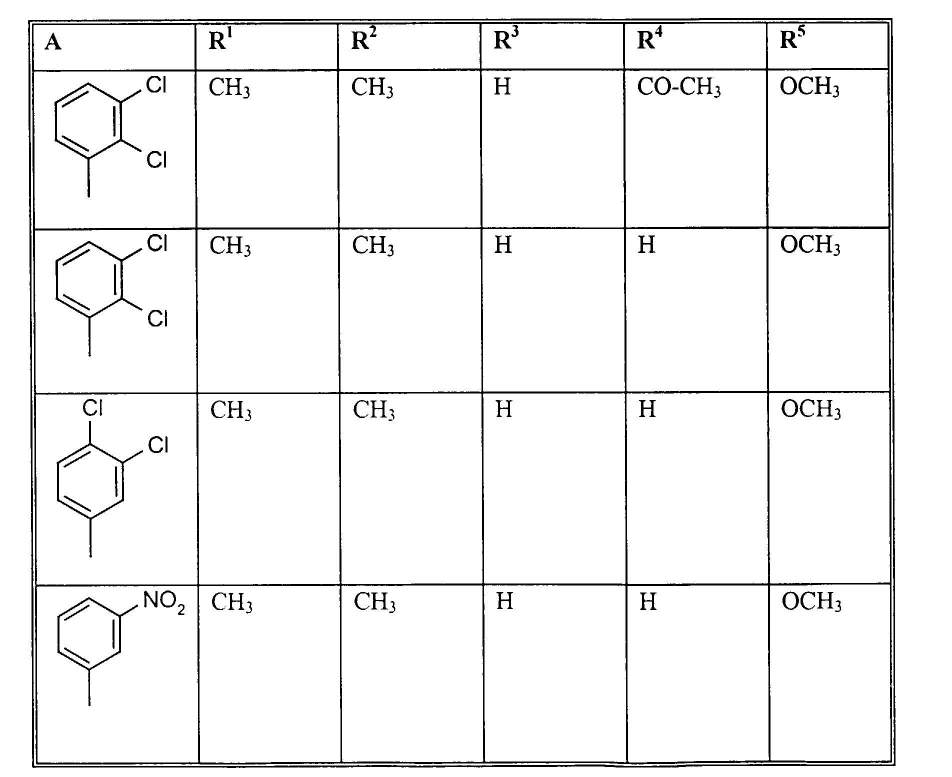

Außerdem wurden Verfahren zur Herstellung der erfindungsgemäßen Verbindungen der allgemeinen Formel (Ia) gefunden, dadurch gekennzeichnet, daß man
[A] Ylidenverbindungen der allgemeinen Formel (II) 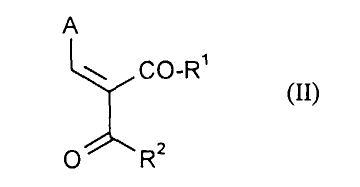 in welcher
   A, R¹ und R² die oben angegebenen Bedeutungen haben,
   im Fall R⁵ = Alkoxy (R^{5'}) mit Verbindungen der allgemeinen Formel (III) und im Fall R⁵ = Alkyl (R^{5''}) mit Verbindungen der allgemeinen Formel (IIIa) 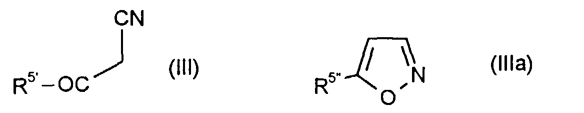 in welchen
   R^{5'} und R^{5"} die oben angegebenen Bedeutungen haben,
   in inerten Lösemitteln, gegebenenfalls in Anwesenheit von Basen, umsetzt,
   oder
[B] Ylidenverbindungen der allgemeinen Formel (IV) 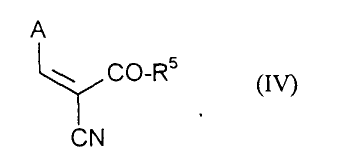 in welcher
   A und R⁵ die oben angegebenen Bedeutungen haben,
   mit Verbindungen der allgemeinen Formel (V) 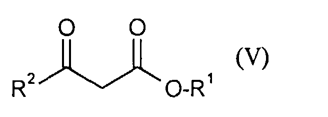 in welcher
   R¹ und R² die oben angegebenen Bedeutungen haben,
   in inerten Lösemitteln und gegebenenfalls in Anwesenheit einer Base umsetzt,
   und im Fall R³ und/oder R⁴ ≠ H eine Alkylierung oder Acylierung nach üblichen Methoden anschließt.

Das erfindungsgemäße Verfahren kann durch folgendes Formelschema beispielhaft erläutert werden:

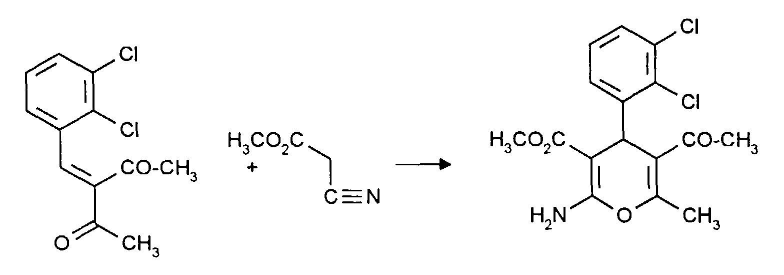

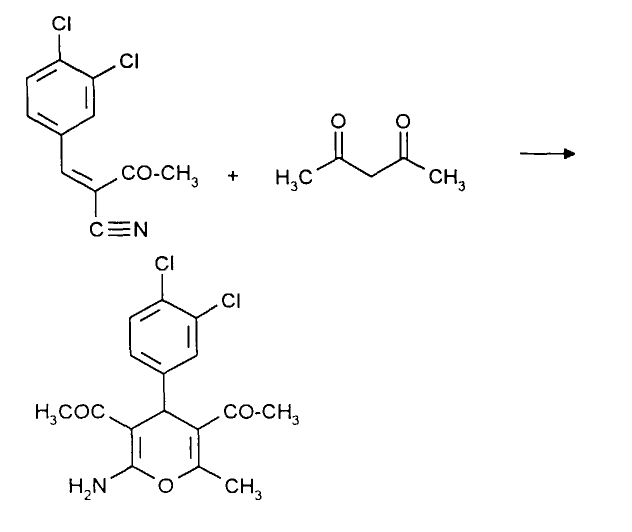

Als Lösemittel eignen sich alle inerten organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether, oder Diethylenglykoldimethylether, Acetonitril, oder Amide wie Hexamethylphosphorsäuretriamid oder Dimethylformamid, oder Essigsäure oder halogenierte Kohlenwasserstoffe wie Methylenchlorid, Tetrachlorkohlenstoff oder Kohlenwasserstoffe wie Benzol oder Toluol, oder Pyridin. Ebenso ist es möglich, Gemische der genannten Lösemittel zu verwenden. Besonders bevorzugt ist Methanol.

Als Basen eignen sich im allgemeinen Alkalihydride- oder -alkoholate, wie beispielsweise Natriumhydrid, Natriumethylat oder Kalium-tert.butylat, oder cyclische Amine, wie beispielsweise Piperidin, Dimethylaminopyridin oder C₁-C₄-Alkylamine, wie beispielsweise Triethylamin. Bevorzugt sind Triethylamin oder Natriumethylat.

Die Base wird im allgemeinen in einer Menge von 1 mol bis 5 mol, bevorzugt von 1 mol bis 2 mol, bezogen auf 1 mol der Verbindungen der allgemeinen Formel (II) eingesetzt.

Die Umsetzungen mit Verbindungen der allgemeinen Formeln (III) und (IIIa) erfolgt im allgemeinen in einem Temperaturbereich von 0°C bis 150°C, bevorzugt von 40°C bis 80°C.

Die Umsetzungen können bei Normaldruck, aber auch bei erhöhtem oder erniedrigtem Druck (z.B. 0,5 bis 3 bar) durchgeführt werden. Im allgemeinen arbeitet man bei Normaldruck.

Enantiomerenreine Formen erhält man z.B., indem man Diastereomerengemische der Verbindungen der allgemeinen Formel (I), in welcher R¹ für einen optisch aktiven Esterrest steht, nach üblicher Methode trennt, anschließend entweder direkt umestert oder zuerst die chiralen Carbonsäuren herstellt und dann durch Veresterung die enantiomerenreinen Verbindungen herstellt.

Die Trennung der Diastereomeren erfolgt im allgemeinen entweder durch fraktionierte Kristallisation, durch Säulenchromatographie oder durch Craig-Verteilung. Welches das optimale Verfahren ist, muß von Fall zu Fall entschieden werden, manchmal ist es auch zweckmäßig, Kombinationen der einzelnen Verfahren zu benutzen. Besonders geeignet ist die Trennung durch Kristallisation oder Craig-Verteilung bzw. eine Kombination beider Verfahren.

Die enantiomerenreinen Verbindungen sind auch zugänglich durch Chromatographie der racemischen Ester auf chiralen Phasen.

Die Verbindungen der allgemeinen Formeln (II), (III), (IIIa), (IV) und (V) sind an sich bekannt oder nach üblichen Methoden herstellbar.

### Rubidium-Efflux aus C6-BU1-Glioma-Zellen

Die Versuche wurden mit geringfügigen Veränderungen entsprechend der von Tas et al. (Neurosci. Lett. 94, 279-284, (1988)) beschriebenen Methode durchgeführt. Dazu werden Ratten C6-BU1-Glioma-Zellen verwendet. Aus den durch Atom Absorptionsspektroskopie erhaltenen Daten wird die durch Ionomycin hervorgerufene Erhöhung des Effluxes über den Basalefflux berechnet und als 100 % gesetzt. Die Stimulationen in Gegenwart von Prüfsubstanzen werden dann auf diesen Wert bezogen.

Zur vorliegenden Erfindung gehören auch pharmazeutische Zubereitungen, die neben inerten, nicht-toxischen, pharmazeutisch geeigneten Hilfs- und Trägerstoffen eine oder mehrere Verbindungen der allgemeinen Formeln (I) / (Ia) enthalten, oder die aus einem oder mehreren Wirkstoffen der Formeln (I) / (Ia) bestehen, sowie Verfahren zur Herstellung dieser Zubereitungen.

Die Wirkstoffe der Formeln (I) / (Ia) sollen in diesen Zubereitungen in einer Konzentration von 0,1 bis 99,5 Gew.-%, bevorzugt von 0,5 bis 95 Gew.-% der Gesamtmischung vorhanden sein.

Neben den Wirkstoffen der Formeln (I) / (Ia) können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden, beispielsweise mit dem oder den Hilfs- oder Trägerstoffen.

Im allgemeinen hat es sich als vorteilhaft erwiesen, den oder die Wirkstoffe der Formeln (I) / (Ia) in Gesamtmengen von etwa 0,01 bis etwa 100 mg/kg, bevorzugt in Gesamtmengen von etwa 1 mg/kg bis 50 mg/kg Körpergewicht je 24 Stunden, gegebenenfalls in Form mehrerer Einzelgaben, zur Erzielung des gewünschten Ergebnisses zu verabreichen.

Es kann aber gegebenenfalls vorteilhaft sein, von den genannten Mengen abzuweichen, und zwar in Abhängigkeit von der Art und vom Körpergewicht des behandelten Objekts, vom individuellen Verhalten gegenüber dem Medikament, der Art und Schwere der Erkrankung, der Art der Zubereitung und Applikation, sowie dem Zeitpunkt bzw. Intervall, zu welchem die Verabreichung erfolgt.

### Beispiel 1

5-Acetyl-2-amino-6-methyl-4-(2,3-dichlorphenyl)-4H-pyran-3-carbonsäuremethylester 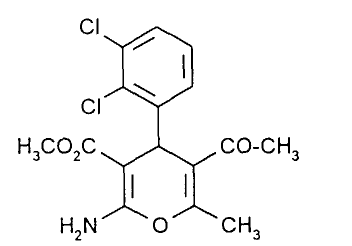
a) Das Gemisch aus 6,0 g (0,034 mol) 2,3-Dichlorbenzaldehyd, 3,4 g (0,034 mol) Pentan-2,4-dion und 3,9 g (0.034 mol) Cyanessigsäuremethylester in 75 ml Methanol wird nach Zugabe von 0,5 ml Eisessig und 0,3 ml Triethylamin 3 h und 40 min zum Rückfluß erhitzt. Nach dem Abkühlen wird der ausgefallene 2-Cyan-3-(2,3-dichlorphenyl)acrylsäuremethylester abfiltriert, das Filtrat im Vakuum eingedampft und der Rückstand (10,5 g) an 300 g Kieselgel mit Toluol/Essigsäureethylester (10:1) chromatographiert.
5,0 g Produkt werden erhalten.
Schmp.: 142-145°C (Kap.) (aus Dichlormethan / Petrolether).
R_{f}-Wert : 0,26 (Toluol/Essigsäureethylester = 3:1)

| | | | | |
|---|---|---|---|---|
| C₁₆H₁₅Cl₂NO₄ | Ber. | C53,9 % | H4,24 % | N3,93 % |
| | Gef. | 54,00 % | H4,18 % | N4,07 % |

b) 5-Acetyl-2-amino-6-methyl-4-(2,3-dichlorphenyl)-4H-pyran-3-carbonsäuremethylester wird ebenfalls erhalten aus den Umsetzungen von 3-Acetyl-4-(2,3-dichlorphenyl)-buten-2-on mit Cyanessigsäuremethylester und 2-Cyan-3-(2,3-dichlorphenyl)acrylsäuremethylester mit Pentan-2,4-dion unter den voranstehend angegebenen Reaktionsbedingungen.

### Beispiel 2

### 5-Acetyl-2-amino-6-methyl-4-(4,3-dichlorphenyl)-4H-pyran-3-carbonsäuremethylester

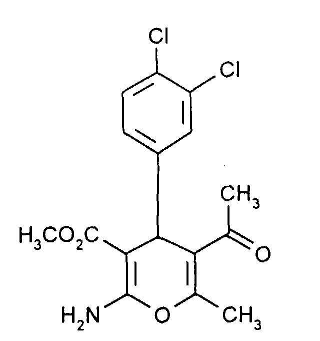

Die Titelverbindung wird in Analogie zu den Vorschriften des Beispiels 1 hergestellt. Schmelzpunkt: 181 bis 185°C (CH₂CH₂/PE)

### Beispiel 3

### 5-Acetyl-2-acetylamino-6-methyl-4-(2,3-dichlorphenyl)-4H-pyran-3-carbonsäuremethylester

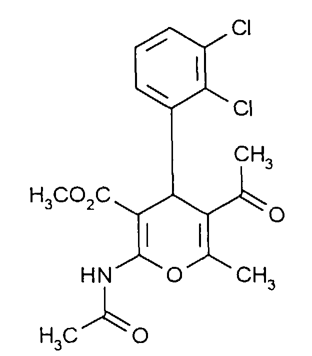

9,2 g (0,026 mmol) 5-Acetyl-2-amino-6-methyl-4-(2,3-dichlorphenyl)-4H-pyran-3-carbonsäuremethylester werden 30 min. in 30 ml Acetanhydrid zum Rückfluß erhitzt. Nach Zugabe von 30 ml Methanol wird das Gemisch im Vakuum eingedampft, und der Rückstand 2 mal mit je 30 ml Toluol im Vakuum abgezogen. Chromatographie des öligen Rückstands (9,1 g) an Kieselgel mit Toluol/Essigsäureethylester (3:1), dessen Struktur durch das H-NMR-Spektrum gesichert ist.

### Beispiel 4

### 5-Acetyl-2-amino-6-methyl-4-(3-nitrophenyl)-4H-pyran-3-carbonsäuremethylester

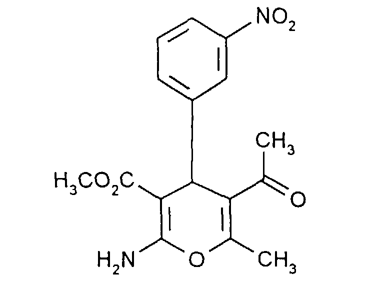

Die Titelverbindung wurde in Analogie zur Vorschrift des Beispiels 1 hergestellt. F°C: 160 bis 163 (CH₂Cl₂/PE)

## Patentansprüche

1. Verwendung von Verbindungen der allgemeinen Formel (I), 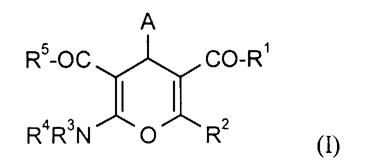 in welcher
A für Aryl mit 6 bis 10 Kohlenstoffatomen oder Pyridyl steht, die gegebenenfalls bis zu 3-fach gleich oder verschieden durch Nitro, Cyano, Phenyl, Halogen, Trifluormethyl oder durch geradkettiges oder verzweigtes Alkylthio oder Alkoxy mit jeweils bis zu 6 Kohlenstoffatomen substituiert sind,
R¹ für geradkettiges oder verzweigtes Alkyl mit bis zu 8 Kohlenstoffatomen steht,
R² für geradkettiges oder verzweigtes Alkyl mit bis zu 6 Kohlenstoffatomen steht,
R³ und R⁴ gleich oder verschieden sind und für Wasserstoff oder für geradkettiges oder verzweigtes Alkyl oder Acyl mit jeweils bis zu 6 Kohlenstoffatomen stehen,
R⁵ für geradkettiges oder verzweigtes Alkyl oder Alkoxy mit jeweils bis zu 8 Kohlenstoffatomen steht,
und/oder deren Salze
zur Herstellung von Arzneimitteln zur Behandlung von Depression, Psychosen, Sichelzellanämie und Ödemen.

2. Verwendung gemäß Anspruch 1,
wobei
A für Phenyl, Naphthyl oder Pyridyl steht, die gegebenenfalls bis zu 3-fach gleich oder verschieden durch Nitro, Cyano, Fluor, Chlor, Brom, Iod, Phenyl, Trifluormethyl oder durch geradkettiges oder verzweigtes Alkylthio oder Alkoxy mit jeweils bis zu 4 Kohlenstoffatomen substituiert sind,
R¹ für geradkettiges oder verzweigtes Alkyl mit bis zu 6 Kohlenstoffatomen steht,
R² für geradkettiges oder verzweigtes Alkyl mit bis zu 4 Kohlenstoffatomen steht,
R³ und R⁴ gleich oder verschieden sind und für Wasserstoff oder für geradkettiges oder für verzweigtes Alkyl oder Acyl mit jeweils bis zu 4 Kohlenstoffatomen stehen,
R⁵ für geradkettiges oder verzweigtes Alkyl oder Alkoxy mit jeweils bis zu 6 Kohlenstoffatomen steht.

3. Verwendung gemäß Anspruch 1,
wobei
A für Phenyl steht, das gegebenenfalls bis zu 3-fach gleich oder verschieden durch Nitro, Cyano, Fluor, Chlor, Brom, Iod, Trifluormethyl oder Methoxy substituiert ist,
R¹ für Methyl oder Ethyl steht,
R² für Methyl oder Ethyl steht,
R³ und R⁴ gleich oder verschieden sind und für Wasserstoff oder für geradkettiges oder verzweigtes Alkyl oder Acyl mit jeweils bis zu 3 Kohlenstoffatomen stehen,
R⁵ für geradkettiges oder verzweigtes Alkyl oder Alkoxy mit jeweils bis zu 4 Kohlenstoffatomen steht.

4. Verbindungen der allgemeinen Formel (Ia) nach Anspruch 1 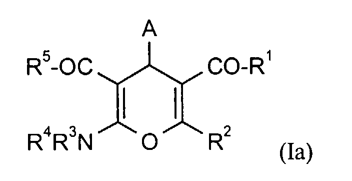 und deren Salze,
mit den in der folgenden Tabelle angegebenen Substituentenbedeutungen:

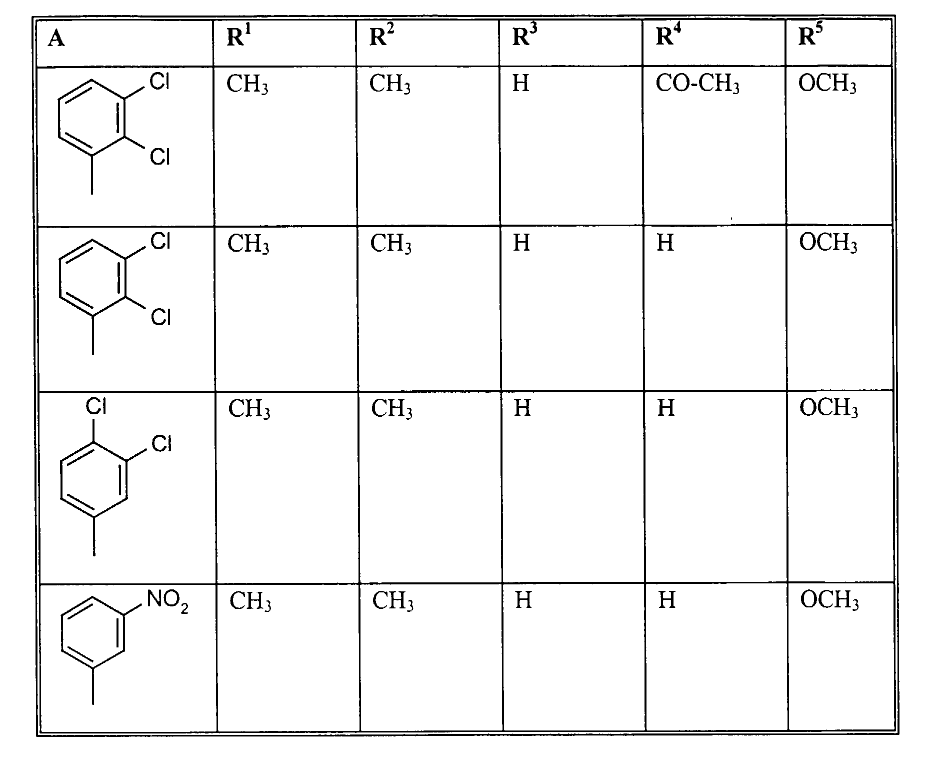

5. Verfahren zur Herstellung der Verbindungen nach Anspruch 4, dadurch gekennzeichnet, daß man
[A] Ylidenverbindungen der allgemeinen Formel (II) 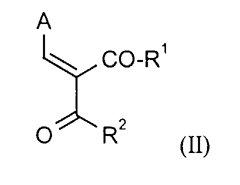 in welcher
A, R¹ und R² die in Anspruch 4 angegebenen Bedeutungen haben,
im Fall R⁵ = Alkoxy (R^{5'}) mit Verbindungen der allgemeinen Formel (III) und im Fall R⁵ = Alkyl (R^{5''}) mit Verbindungen der allgemeinen Formel (IIIa) 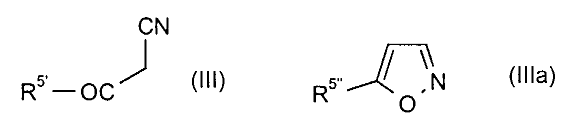 in welchen
R^{5'} und R^{5''} die oben angegebenen Bedeutungen haben,
in inerten Lösemitteln, gegebenenfalls in Anwesenheit von Basen, umsetzt,
oder
[B] Ylidenverbindungen der allgemeinen Formel (IV) 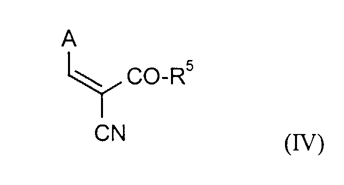 in welcher
A und R⁵ die in Anspnich 4 angegebenen Bedeutungen haben,
mit Verbindungen der allgemeinen Formel (V) 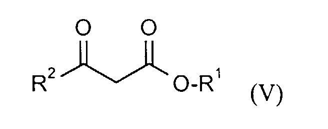 in welcher
R¹ und R² die in Anspruch 4 angegebenen Bedeutungen haben,
in inerten Lösemitteln und gegebenenfalls in Anwesenheit einer Base umsetzt,
und im Fall R³ und/oder R⁴ ≠ H eine Alkylierung oder Acylierung nach üblichen Methoden anschließt.

6. Arzneimittel enthaltend mindestens ein acyl-substituiertes Aminopyran nach Anspruch 4 und/oder dessen Salze.

7. Verwendung von acyl-substituierten Aminopyranen nach Anspruch 4 und/oder deren Salze zur Herstellung von Arzneimitteln zur Behandlung von Depression, Psychosen, Sichelzellanämie und Ödemen.

## Claims

1. Use of compounds of the general formula (I) 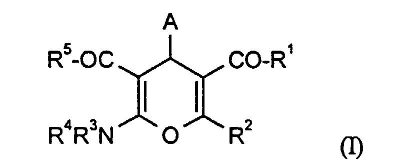 in which
A represents aryl having 6 to 10 carbon atoms or pyridyl, each of which is optionally substituted up to 3 times by identical or different nitro, cyano, phenyl, halogen or trifluoromethyl or by straight-chain or branched alkylthio or alkoxy each having up to 6 carbon atoms,
R¹ represents straight-chain or branched alkyl having up to 8 carbon atoms,
R² represents straight-chain or branched alkyl having up to 6 carbon atoms,
R³ and R⁴ are identical or different and represent hydrogen or straight-chain or branched alkyl or acyl each having up to 6 carbon atoms,
R⁵ represents straight-chain or branched alkyl or alkoxy each having up to 8 carbon atoms,
and/or their salts
for the production of medicaments for the treatment of depression, psychoses, sickle-cell anaemia and oedemas.

2. Use according to Claim 1, in which
A represents phenyl, naphthyl or pyridyl, each of which is optionally substituted up to 3 times by identical or different nitro, cyano, fluorine, chlorine, bromine, iodine, phenyl or trifluoromethyl or by straight-chain or branched alkylthio or alkoxy each having up to 4 carbon atoms,
R¹ represents straight-chain or branched alkyl having up to 6 carbon atoms,
R² represents straight-chain or branched alkyl having up to 4 carbon atoms,
R³ and R⁴ are identical or different and represent hydrogen or straight-chain or branched alkyl or acyl each having up to 4 carbon atoms,
R⁵ represents straight-chain or branched alkyl or alkoxy each having up to 6 carbon atoms.

3. Use according to Claim 1, in which
A represents phenyl which is optionally substituted up to 3 times by identical or different nitro, cyano, fluorine, chlorine, bromine, iodine, trifluoromethyl or methoxy,
R¹ is methyl or ethyl,
R² is methyl or ethyl,
R³ and R⁴ are identical or different and represent hydrogen or straight-chain or branched alkyl or acyl each having up to 3 carbon atoms,
R⁵ represents straight-chain or branched alkyl or alkoxy each having up to 4 carbon atoms.

4. Compounds of the general formula (Ia) according to Claim 1 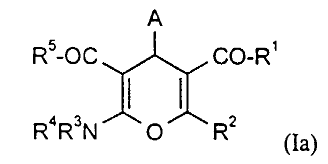 and their salts, having the substituent meanings indicated in the following table:

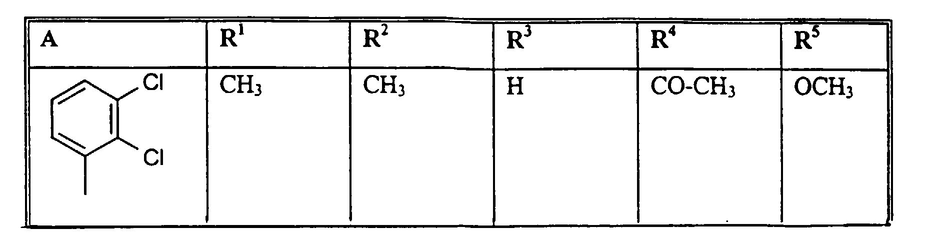

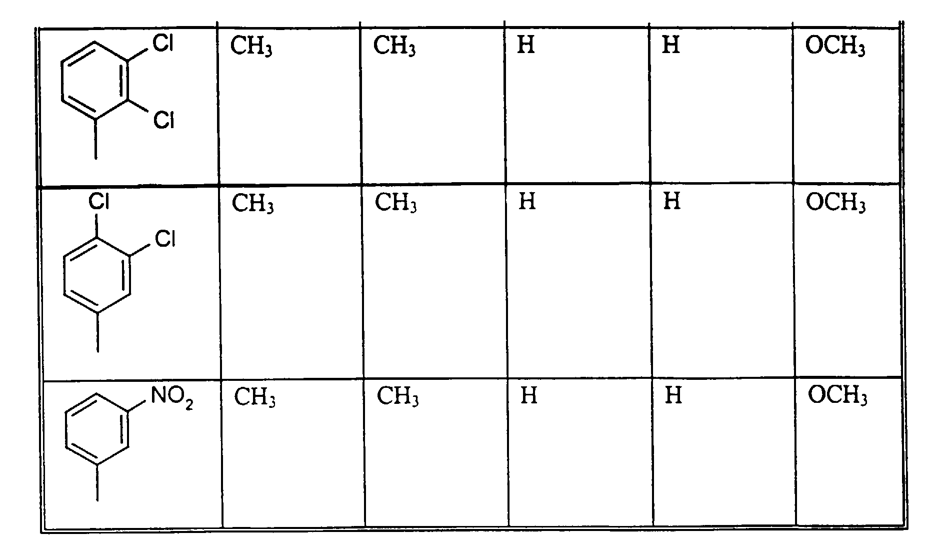

5. Process for the preparation of the compounds according to Claim 4, characterized in that
[A] ylidene compounds of the general formula (II) 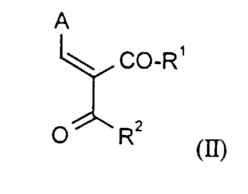 in which
A, R¹ and R² have the meanings indicated in Claim 4,
are reacted, if R⁵ = alkoxy (R^{5'}), with compounds of the general formula (III) and, if R⁵ = alkyl (R^{5"}), with compounds of the general formula (IIIa) 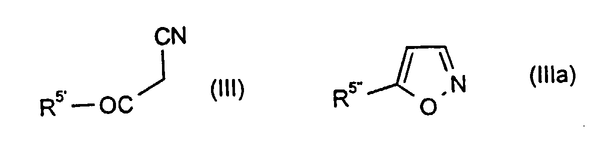 in which
R^{5'} and R^{5"} have the meanings indicated above,
in inert solvents, if appropriate in the presence of bases,
or
[B] ylidene compounds of the general formula (IV) 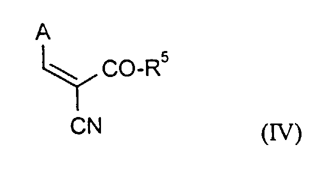 in which
A and R⁵ have the meanings indicated in Claim 4,
are reacted with compounds of the general formula (V) 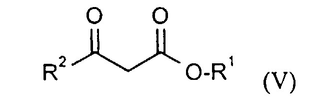 in which
R¹ and R² have the meanings indicated in Claim 4,
in inert solvents and if appropriate in the presence of a base, and if R³ and/or R⁴ ≠ H an alkylation or acylation according to customary methods follows.

6. Medicaments comprising at least one acyl-substituted aminopyran according to Claim 4 and/or its salts.

7. Use of acyl-substituted aminopyrans according to Claim 4 and/or their salts for the production of medicaments for the treatment of depression, psychoses, sickle-cell anaemia and oedemas.

## Revendications

1. Utilisation de composés répondant à la formule générale (I) 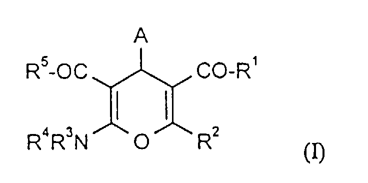 dans laquelle
A représente un groupe aryle contenant de 6 à 10 atomes de carbone ou un groupe pyridyle, ces groupes portant le cas échéant jusqu'à trois substituants identiques ou différents nitro, cyano, phényle, halogéno, trifluorométhyle ou encore alkylthio ou alcoxy à chaînes droites ou ramifiées contenant respectivement jusqu'à 6 atomes de carbone,
R¹ représente un groupe alkyle à chaîne droite ou ramifiée contenant jusqu'à 8 atomes de carbone,
R² représente un groupe alkyle à chaîne droite ou ramifiée contenant jusqu'à 6 atomes de carbone,
R³ et R⁴ sont identiques ou différents et représentent un atome d'hydrogène ou encore un groupe alkyle ou un groupe acyle à chaîne droite ou ramifiée contenant respectivement jusqu'à 6 atomes de carbone,
R⁵ représente un groupe alkyle ou un groupe alcoxy à chaîne droite ou ramifiée contenant respectivement jusqu'à 8 atomes de carbone,
et/ou de leurs sels,
pour la préparation de médicaments pour le traitement de la dépression, des psychoses, de l'anémie drépanocytaire et des oedèmes.

2. Utilisation selon la revendication 1, dans laquelle
A représente un groupe phényle, un groupe naphtyle ou un groupe pyridyle qui portent le cas échéant jusqu'à trois substituants identiques ou différents nitro, cyano, fluoro, chloro, bromo, iodo, phényle, trifluorométhyle ou encore alkylthio ou alcoxy à chaînes droites ou ramifiées contenant respectivement jusqu'à 4 atomes de carbone,
R¹ représente un groupe alkyle à chaîne droite ou ramifiée contenant jusqu'à 6 atomes de carbone,
R² représente un groupe alkyle à chaîne droite ou ramifiée contenant jusqu'à 4 atomes de carbone,
R³ et R⁴ sont identiques ou différents et représentent un atome d'hydrogène ou encore un groupe alkyle ou un groupe acyle à chaîne droite ou ramifiée contenant respectivement jusqu'à 4 atomes de carbone,
R⁵ représente un groupe alkyle ou un groupe alcoxy à chaîne droite ou ramifiée contenant respectivement jusqu'à 6 atomes de carbone.

3. Utilisation selon la revendication 1, dans laquelle
A représente un groupe phényle qui porte le cas échéant jusqu'à trois substituants identiques ou différents nitro, cyano, fluoro, chloro, bromo, iodo, trifluorométhyle ou méthoxy,
R¹ représente un groupe méthyle ou un groupe éthyle,
R² représente un groupe méthyle ou un groupe éthyle,
R³ et R⁴ sont identiques ou différents et représentent un atome d'hydrogène ou encore un groupe alkyle ou un groupe acyle à chaîne droite ou ramifiée contenant respectivement jusqu'à 3 atomes de carbone,
R⁵ représente un groupe alkyle ou un groupe alcoxy à chaîne droite ou ramifiée contenant respectivement jusqu'à 4 atomes de carbone.

4. Composés répondant à la formule générale (Ia) selon la revendication 1 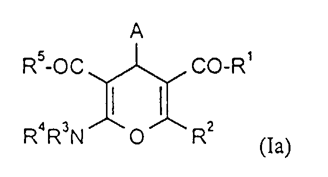 ainsi que leurs sels,
avec les significations des substituants indiquées dans le tableau ci-après:

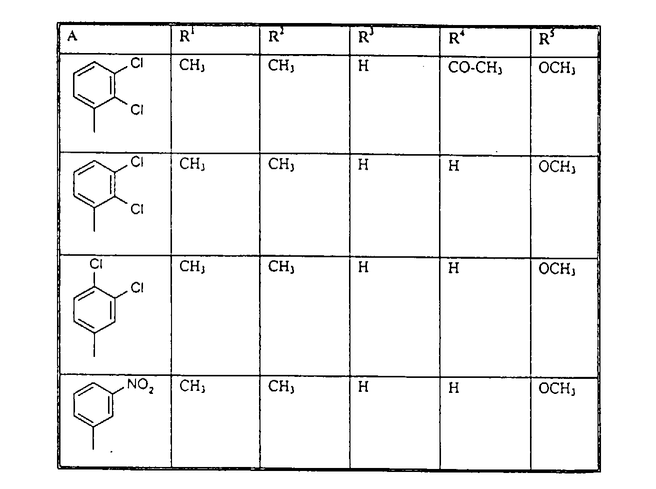

5. Procédé pour la préparation des composés selon la revendication 4, caractérisé en ce qu'on fait réagir
[A] des composés d'ylidène répondant à la formule générale (II) 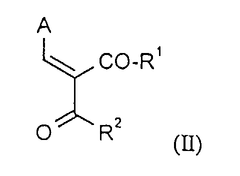 dans laquelle
A, R¹ et R² ont les significations indiquées à la revendication 4,
dans le cas où R⁵ représente un groupe alcoxy (R^{5'}) avec des composés répondant à la formule générale (III) et dans le cas où R⁵ représente un groupe alkyle (R^{5"}) avec des composés répondant à la formule générale (IIIa) 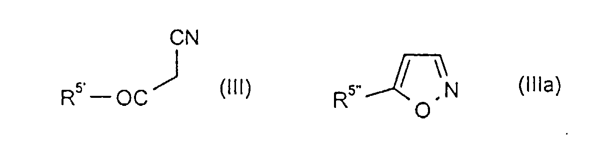 dans lesquelles
R^{5'} et R^{5"} ont les significations indiquées ci-dessus,
dans des solvants inertes, le cas échéant en présence de bases,
ou
[B] des composés d'ylidène répondant à la formule générale (IV) 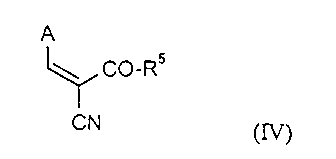 dans laquelle
A et R⁵ ont les significations indiquées à la revendication 4,
avec des composés répondant à la formule générale (V) 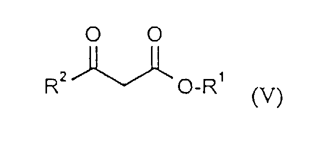 dans laquelle
R¹ et R² ont les significations indiquées à la revendication 4,
dans des solvants inertes et le cas échéant en présence d'une base, et dans le cas où R³ et/ou R⁴ ne représentent pas un atome d'hydrogène, on fait suivre cette mise en réaction d'une alkylation ou d'une acylation conformément à des procédés habituels.

6. Médicament contenant au moins un aminopyranne portant un ou plusieurs substituants acyle selon la revendication 4 et/ou ses sels.

7. Utilisation d'aminopyrannes portant un ou plusieurs substituants acyle selon la revendication 4 et/ou de leurs sels pour la préparation de médicaments destinés au traitement de la dépression, des psychoses, de l'anémie drépanocytaire et des oedèmes.
